# EUROPEAN PATENT SPECIFICATION

(11) **EP 3 934 643 B1**
(45) Date of publication and mention of the grant of the patent: **17.06.2026**
(21) Application number: 20766548.0
(22) Date of filing: 02.03.2020
(51) Int. Cl.: A61K 31/4178, B05B 17/06, A61K 31/4168, A61K 9/00, A61P 27/02, B05B 17/00

(54) **COMPOSITION COMPRISING PILOCARPINE FOR USE IN THE TREATMENT OF PRESBYOPIA BEING DELIVERED TO THE EYE AS A MICRO-DOSE STREAM OF DROPLETS**
ZUSAMMENSETZUNG, DIE PILOCARPIN ZUR BEHANDLUNG VON PRESBYOPIE ENTHÄLT UND DEM AUGE ALS MIKRODOSIERTER TRÖPFELSTROM ZUGEFÜHRT WIRD
COMPOSITION CONTENANT DE LA PILOCARPINE POUR LE TRAITEMENT DE LA PRESBYOPIE, ADMINISTRÉE À L'OEIL SOUS FORME DE MICRODOSES DE GOUTTELETTES

(30) Priority: 04.03.2019 US 201962813608 P
(43) Date of publication of application: 12.01.2022
(73) Proprietor: Hyperion DeFi, Inc., New York, NY 10017 (US)
(72) Inventor: IANCHULEV, Tsontcho, New York, New York 10017 (US); CLAUSON, Luke, Reno, Nevada 89521 (US)
(74) Representative: HGF
(86) International application number: PCT/US2020/020656
(87) International publication number: WO 2020/180793

(56) References cited:
- WO-A1-2010/135731
- WO-A1-2010/135731
- WO-A1-2018/227190
- WO-A1-2018/227190
- US-A1- 2005 279 369
- US-A1- 2012 143 152
- US-A1- 2014 171 490
- US-A1- 2015 174 105
- US-A1- 2017 136 484
- ABRAMSON D H ET AL: "PILOCARPINE IN THE PRESBYOPE. DEMONSTRATION OF AN EFFECT ON THE ANTERIOR CHAMBER AND LENS THICKNESS", ARCHIVES OF OPHTHALMOLOGY, AMERICAN MEDICAL ASSOCIATION, UNITED STATES, vol. 89, no. 2, 1 February 1973 (1973-02-01), pages 100 - 102, XP000866126, ISSN: 0003-9950
- ANONYMOUS: "Addressing presbyopia pharmacologically", OPHTHALMOLOGY TIMES, 24 April 2012 (2012-04-24), Internet, pages 1 - 2, XP055973782, Retrieved from the Internet <URL:https://www.ophthalmologytimes.com/view/addressing-presbyopia-pharmacologically> [retrieved on 20221021]

## Description

### PRIORITY

This application claims the benefit under 35 U.S.C. §119(e) of U.S. Provisional Patent Application Serial No. 62/813,608, entitled "METHODS AND DEVICES FOR DELIVERING PILOCARPINE TO THE EYE AS A MICRO-DOSE STREAM OF DROPLETS," filed on March 4, 2019.

### FIELD OF THE INVENTION

The present disclosure relates to compositions for use according to the claims. Also disclosed are droplet delivery devices, methods of administration and uses thereof, specifically for the administration of droplets to the eye.

### BACKGROUND OF THE INVENTION

Presbyopia typically affects patients later in life, e.g., after about the age of 40, and is characterized by accommodative vision loss that leads a decrease in quality of life. The current treatment for presbyopia is generally reading or bifocal glasses. There is significant need for safe, non-invasive and reversible treatment to augment reading vision and accommodative capacity. In this regard, there is currently no pharmacologic treatment approved. However, in recent years, pharmacological solutions to presbyopia have been proposed. Exemplary pharmaceutical approaches, as reported in the literature are provided in the table below, including examples of the administration of pilocarpine cocktails and combinations for the treatment of presbyopia.

| **API** | **Results** | **Reference/Notes** |
|---|---|---|
| **Brimonidine 0.2%** | Near VA 4 line improvement | **Eye Contact Lens. 2015;41(5):323-7** : 48 naturally emmetropic and presbyopic subjects aged between 43 and 56 years, which was aimed at evaluating the efficacy of instilling carbachol 2.25% with brimonidine 0.2% eye drops monocularly once daily for 3 months |
| **Parasym + NSAID** | Near VA 0.35→0.649 | **Ref: http://ophthalmologytimes.modernmedicine.com/ophth almologytimes/news/ simple-solution-presbyopia.** |
| | | parasympathomimetic agent with an NSAID in an oil-based |
| | | formulation 81 patients from 42 to 74 years |
| **Pilocarpine 0.24% Cocktail** | Near VA 2- 3 line improvement J 3.5 →J 1.5 | Ophthalmol Ther. 2016;5(1):63-73 Cocktail: Pilocarpine 0.247%, Phenylephrine 0.78%, Polyethyleneglycol 0.09%, Nepafenac 0.023%, Pheniramine 0.034%, and Naphazoline 0.003% 14 presbyopic subjects aged 41 to 55 years |
| **pilocarpine 1% and diclofenac 0.1%** | | Med Hypothesis Discov Innov Ophthalmol. 2012;1(1):3-5**.** |
| | | 100 patients between 45 and 50 years |

A typical medical droplet as dispensed by an eye dropper bottle can vary, depending on the viscosity and surface tension of the fluid. In order to control the amount of active ingredient that is administered in a single droplet, the concentration of the active ingredient is adjusted by volume. Once the concentration is defined, a correct dosage may require one drop or more. However, since the human eye can typically retain only 7 µl of fluid at a time, even a single medical droplet can result in overflow and loss of part of the medication from the eye. Multiple drop dosage often compounds the problem of medication retention in the eye. Subjects will typically administer all droplets required for a dosage in one sitting, which exacerbates the problem and can result in 50 to 90% of the medication overflowing and leaking out of the eye.

Given the above and other limitations of current ophthalmic delivery, a need exists for an efficient delivery system for solutions to the eye, including solutions containing medicaments such as pilocarpine.

WO 2010/135731 A1 describes the use of one or more parasympathomimetic drugs or a cholinesterase inhibitor in combination with one or more alpha agonists or antagonist to create optically beneficial miosis to, for example, temporarily treat presbyopia.

US 2015/174105 A1 describes an ophthalmic composition for the correction of presbyopia, wherein the composition comprises of 2% Pilocarpine and bromfenac.

Abramson D H et al. "Pilocarpine in the presbyope. Demonstration of an effect on the anterior chamber and lens thickness", Archives of Ophthalmology, American Medical Association, United States, (19730201), vol. 89, no. 2, pages 100 - 102, describes the effect of pilocarpine on changes in the anterior chamber depth and lens thickness.

Anonymous, "Addressing presbyopia pharmacologically", Ophthalmology Times, Internet, (20120424), pages 1 - 2, URL: https://www.ophthalmologytimes.com/view/addressing-presbyopia-pharmacologically, describes monocular treatment of the non-dominant eye by instillation of pilocarpine or carbachol, both cholinergic agonists, and bromonidine, an alpha agonist that can prolong the effect of cholinergic agonists, as a potential approach to treating presbyopia.

US 2012/143152 A1 describes a method of delivering safe, suitable, and repeatable dosages to a subject for topical, oral, nasal, or pulmonary use and a device for droplet ejection which includes a fluid delivery system capable of delivering a defined volume of the fluid in the form of droplets having properties that afford adequate and repeatable high percentage deposition upon application.

WO 2018/227190 A1 describes methods and devices for handling a fluid and delivering the fluid to the eye.

### SUMMARY OF THE INVENTION

Provided herein is a composition comprising pilocarpine for use in the treatment of presbyopia in an eye of a subject, wherein the composition is delivered to the eye as a micro-dose stream of droplets, wherein:
the micro-dose stream of droplets including the composition comprising pilocarpine is generated via a piezoelectric droplet delivery device, wherein the micro-dose stream of droplets has an average ejected droplet diameter greater than 15 microns.

Also disclosed, but not claimed, is a method for delivering a composition comprising pilocarpine to an eye of a subject in need thereof as a micro-dose stream of droplets. The method generally includes, (a) generating a micro-dose stream of droplets including the composition comprising pilocarpine via a piezoelectric droplet delivery device, wherein the micro-dose stream of droplets has an average ejected droplet diameter greater than 15 microns; and (b) delivering the micro-dose stream of droplets to the eye of said subject. In certain embodiments of the invention, the composition for use treats or alleviates one or more symptoms of presbyopia, in particular presbyopia in adults 40 years of age or older, in the subject in need thereof.

In certain embodiments, the composition comprises a relatively high concentration of pilocarpine as a single active agent. In certain embodiments, the composition comprising pilocarpine does not include pain relieving medications including non-steroidal anti-inflammatory drugs (NSAIDs, such as nepafenac or diclofenac), antihistamine (such as pheniramine or decongestant medications (such as oxymetazoline, phenylephrine, or naphazoline), or other active agent. In this regard, in certain embodiments, pilocarpine is the only active pharmaceutical ingredient present in the composition delivered to the eye of the subject.

In certain aspects, the low volume micro-dose of a composition comprises pilocarpine at a concentration of at least about 0.5% by weight, at least about 1% by weight, at least about 2 % by weight, at least about 3% by weight, at least about 4% by weight, about 1% by weight, about 2% by weight, about 3% by weight, about 4% by weight, from about 0.5 to about 1% by weight, from about 0.5 to about 2% by weight, from about 0.5 to about 3% by weight, from about 0.5 to about 4% by weight, from about 1% to about 2% by weight, from about 1% to about 3% by weight, from about 1% to about 4% by weight, etc.

In certain aspects, the piezoelectric droplet delivery device comprises an ejector mechanism, the ejector mechanism comprising a generator plate and a piezoelectric actuator, wherein the generator plate includes a plurality of openings formed through its thickness; and wherein the piezoelectric actuator is operable to directly or indirectly oscillate the generator plate, at a frequency to generate a directed stream of droplets of said low dosage volume medicament composition.

In certain embodiments, the micro-dose stream of droplets delivered to the eye of the subject is less than 13 microliters, less than 10 microliters, less than 5 microliters, 3-7 microliters, etc. In other embodiments, the micro-dose stream of droplets has an average initial ejecting velocity about of at least about 3 m/s, 4 m/s to about 12 m/s, etc. In other embodiments, the micro-dose stream of droplets has an average ejected droplet diameter of greater than 15 microns, 20-60 microns, etc. In yet other embodiments, the micro-dose stream of droplets is delivered to the eye of the subject in less than 80 ms.

In yet other aspects, the piezoelectric droplet delivery device is oriented within 25° of horizontal during generation and delivery of the micro-dose stream of droplets.

These and other features will become apparent from the following description of the preferred embodiments, claims and drawings.

### DETAILED DESCRIPTION

The present invention is defined by the claims. Any subject matter falling outside the scope of the claims is provided for information purposes only. Any references in the description to methods for treatment of the human or animal body by therapy refer to the compositions of the present invention for use in methods for the treatment of the human or animal body by therapy.

The present disclosure provides compositions for use as defined in the claims which improve upon prior art methods and devices for delivering pilocarpine to the eye. Specifically, the present disclosure utilizes relatively high concentrations of pilocarpine in the solution to be administered, contrary to current trends, but delivers a much smaller volume/dose to a subject, which thereby reduces, and even prevents, excess fluid from being unabsorbed by the target tissues. Maintaining higher concentrations of pilocarpine in the solution to be administered may also reduce or avoid issues with the instability of lower concentration solutions.

Moreover, without intending to be limited by theory, previous attempts to utilize pilocarpine have focused on compositions comprising cocktails and combinations of active agents to mitigate potential side effects of pilocarpine. Pilocarpine, while being effective to induce miosis, can also cause accommodative spasm and brow ache as a result of muscarinic stimulation of the ciliary muscle, which can thereby result in myopic shift. Pilocarpine can also cause chronic anterior segment inflammations, synechiae, and pigment dispersion. As such, previous approaches have utilized additional active agents to counteract ciliary muscle spasm, vascular congestion and hyperemia induced by pilocarpine in an effort to avoid an excess of pupil constriction. For instance, NSAIDs may be co-administered to mitigate inflammation, but these co-agents do not address issues with myopic shift. In accordance with aspects of the present disclosure has unexpectedly found that relatively high concentrations of pilocarpine in solution can be administration as small volume micro-doses without the need for cocktail or combination administrations.

In certain embodiments, the composition for use may comprise a relatively high concentration of pilocarpine as a single active agent. In certain embodiments, the composition comprising pilocarpine does not include pain relieving medications including non-steroidal anti-inflammatory drugs (NSAIDs, such as nepafenac or diclofenac), antihistamine (such as pheniramine or decongestant medications (such as oxymetazoline, phenylephrine, or naphazoline), or other active agent. In this regard, in certain embodiments, pilocarpine is the only active pharmaceutical ingredient present in the composition delivered to the eye of the subject.

Also disclosed, but not claimed, is a method for delivering a composition comprising pilocarpine to an eye of a subject in need thereof as a micro-dose stream of droplets. The method generally includes, (a) generating a micro-dose stream of droplets including the composition comprising pilocarpine via a piezoelectric droplet delivery device, wherein the micro-dose stream of droplets has an average ejected droplet diameter greater than 15 microns; and (b) delivering the micro-dose stream of droplets to the eye of said subject. In certain embodiments of the invention, the composition for use treats or alleviates one or more symptoms of presbyopia, in particular presbyopia in adults 40 years of age or older, in the subject in need thereof. In some embodiments of the invention, the composition for use is for the temporary treatment or alleviation of one or more symptoms of presbyopia. For instance, the composition for use may provide for the temporary improvement of functional near vision in presbyopia as compared to a placebo.

In certain aspects, the composition for use utilizes relatively high concentrations of pilocarpine to avoid the stability problems of low concentration formulations currently favored.

In certain embodiments, the disclosure provides a piezoelectric droplet delivery device as defined in the claims, configured to achieve micro-dosing that is many times more precise than conventional eye droppers. In certain aspects, the micro-dosing delivers doses of 6-8 µL in a targeted manner, directly coating the corneal surface (where 80% of intraocular drug penetration occurs) rather than the conjunctiva of the eye of the subject.

Without intending to be limited by theory, it is believed that focusing a significant portion (i.e., over 50%, 60%, 70%, 80%, etc.) of the active agent to be administered to the eye of the subject directly to the corneal surface rather than the conjunctiva reduces collateral tissue exposure. In this regard, it is believed that administration directly to the corneal surface will reduce greater than 75% of the active agent's potential systemic exposure, to thereby reduce toxicity and result in potentially gentler and more tolerable treatments. In other aspects, the micro-therapeutic approach of the disclosure also reduces waste associated with conventional eye droppers.

In yet other aspects, advantages of the micro-dosing approach of the disclosure includes:
**Dose reduction:** micro-dosing achieves precise volumetric control at the microliter level to deliver 6-8 µL, which is the physiologic capacity of the tear film, e.g., as compared to an eye dropper pipette macro-dose of 30-50 µL which results in overdosing, ocular toxicity and systemic leaching into the plasma.
**Targeted dose instillation:** the piezoelectric droplet delivery device described herein allows for targeted delivery to the ocular surface and cornea, avoiding the conjunctival cul-de-sac. The droplet micro-jet created by the piezoelectric vibrations is columnated and focused to provide accurate delivery to the corneal surface where the majority of ocular penetration occurs. Additionally, in certain embodiments, the device may include an LED targeting mechanism to allow proper positioning and objective alignment.
**Speed of delivery:** unlike a simple aerosolized mechanism, the piezoelectric droplet delivery device provides micro-droplet ejection control that creates a fast and targeted micro-jet delivery that provides ejected droplets to the ocular surface in less than 80 milliseconds, beating the eye's 100 millisecond blink reflex.
**Smart electronics:** in certain embodiments, the piezoelectric droplet delivery device includes smart electronics and mobile e-health technology designed to track when a patient administers treatment. This enables physicians to monitor patient compliance accurately. In certain aspects, this technology will improve compliance and chronic disease management by empowering patients and physicians with access to dynamic, real time monitoring and compliance data for a more intelligent and personalized therapeutic paradigm.

In other aspects, the disclosure generally relates to piezoelectric droplet delivery devices as defined in the claims, useful, *e.g.,* in the delivery of a directed stream of droplets for ophthalmic use, more particularly, for use in the delivery of ophthalmic fluid to the eye. Droplets may be formed by an ejector mechanism from fluid contained in a reservoir coupled to the ejector mechanism. Except as otherwise described herein, the ejector mechanism and reservoir may be disposable or reusable, and the components may be packaged in a housing of an ejector device.

In certain embodiments, devices are provided and methods are described for the reproducible delivering a therapeutically effective low volume micro-dose of a composition to a desired target (e.g., an eye of a subject in need thereof, as compared to standard eye dropper use and dosage volume). In certain aspects, the low volume micro-dose of a composition comprises pilocarpine at a concentration of at least about 0.5% by weight, at least about 0.7% by weight, at least about 0.8 % by weight, at least about 1% by weight, at least about 2% by weight, at least about 3% by weight, at least about 4% by weight, etc. In certain embodiments, the composition comprises, about 1%, about 2%, about 3%, about 4%, from about 1% to about 4%, etc. of pilocarpine, by weight. In certain aspects, the composition for use is for the treatment or alleviation of one or more symptoms of presbyopia, in particular presbyopia in adults 40 years of age or older.

In certain aspects, the therapeutically effective low volume micro-dose of a composition may be delivered to an eye at, *e.g.,* 3/4, 1/2, 1/4, 1/6, 1/8, (e.g., ~0.02-0.75) etc. of the volume of a standard eyedropper volume. By way of example, in certain embodiments, from 0.5 µl - 15 µl, from 3 µl - 8 µl, from 7 µl - 8 µl, less than 15 µl, less than 13 µl, less than 10 µl, less than 8 µl, less than 5 µl, etc. of a micro-dose of a composition may be delivered to the eye of a subject, as compared to approximately 25 µl to approximately 70 µl by way of a standard eye dropper, while obtaining equivalent or improved therapeutic efficacy.

Dosing strategies also may incorporate various approaches to initiating treatment, stopping treatment, switching treatment and responding to different subject states. Examples of dosing modes or strategies include once-a-day dosing, twice-a-day dosing, three times-a-day dosing, continuous dosing, bolus dosing, weekly dosing, monthly dosing, taper dosing, need-based dosing, and feedback dosing by a physician, provider, subject, or family. In addition, dosing schemes may include dosing per eye, as needed. The clinical scenarios where these can be employed include chronic disease, disease exacerbation, need for suppression treatment, need for recurrence treatment, or state of treatment like medicament tolerance.

Also disclosed, but not claimed, is a method of delivering a therapeutically effective low volume micro-dose of a composition to an eye of a subject in need thereof, e.g., for the treatment or alleviation of one or more symptoms of presbyopia, in particular presbyopia in adults 40 years of age or older, as compared to dosage volume of a standard eye dropper, the method comprising: (a) generating a directed stream of droplets of the low volume micro-dose of a composition, wherein the droplets have a desired average drop size and average initial ejecting velocity; and (b) delivering a therapeutically effective amount of the droplets of the low volume micro-dose of the composition to the eye of the subject, wherein the droplets deliver a desired percentage of the ejected mass of the droplets to the eye.

Again, in certain aspects, the low volume micro-dose of a composition comprises pilocarpine at a concentration of at least about 0.5% by weight, at least about 1% by weight, at least about 2 % by weight, at least about 3% by weight, at least about 4% by weight, about 1% by weight, about 2% by weight, about 3% by weight, about 4% by weight, from about 0.5 to about 1% by weight, from about 0.5 to about 2% by weight, from about 0.5 to about 3% by weight, from about 0.5 to about 4% by weight, from about 1% to about 2% by weight, from about 1% to about 3% by weight, from about 1% to about 4% by weight, etc.

In certain embodiments, the composition for use comprises a relatively high concentration of pilocarpine as a single active agent. In certain embodiments, the composition comprising pilocarpine does not include pain relieving medications including non-steroidal anti-inflammatory drugs (NSAIDs, such as nepafenac or diclofenac), antihistamine (such as pheniramine or decongestant medications (such as oxymetazoline, phenylephrine, or naphazoline), or other active agent. In this regard, in certain embodiments, pilocarpine is the only active pharmaceutical ingredient present in the composition delivered to the eye of the subject.

In certain embodiments, are compositions for use in the treatment and/or alleviation of one or more symptoms of presbyopia in a subject in need thereof. In certain embodiments, are compositions for use in the treatment and/or alleviation of one or more symptoms of presbyopia comprises increased accommodation of vision in under about 30 minutes, under about 1 hour, under about 2 hours, under about 3 hours, under about 4 hours, under about 5 hours, under about 6 hours, etc., from initial administration of the stream of droplets. In certain embodiments, the accommodation of vision has a duration of effect of at least 2 hours, at least 4 hours, at least 6 hours, etc. In certain embodiments, a 10% increase, 20% increase, 25% increase, 30% increase, 40% increase 50% increase, etc., in accommodation capacity is obtained. In certain embodiments, an accommodation of vision of a % 2 line increase in Uncorrected Visual Acuity (UCVA) is obtained.

Devices capable of providing and delivering therapeutically effective low volume micro-dose of the compositions to the eye are described herein. By way of example, the droplet delivery device may include a housing that comprises a fluid reservoir in fluid communication with an ejector mechanism. The directed stream of droplets may be generated via the ejector mechanism, the ejector mechanism comprising a generator plate and a piezoelectric actuator, wherein the generator plate includes a plurality of openings formed through its thickness. The piezoelectric actuator may be operable to directly or indirectly oscillate the generator plate, at a frequency to generate a directed stream of droplets of the low volume micro-dosing composition.

Without limitation, the droplet delivery device may be as described in US 8,684,980 or WO 2018/227190.

In one embodiment, the device further includes a fluid enclosure system to facilitate ejection of the stream of droplets. In such an embodiment, the fluid to be delivered to the eye is contained in an enclosure which holds the fluid to be dispensed in a chamber defined by the enclosure. The enclosure holds the dose of fluid in proximity to the openings of the generator plate of the ejector mechanism so that the fluid may be ejected in a short amount of time and with little residual volume left.

The enclosure has a lip positioned adjacent the generator plate. The lip may be unattached to the generate plate, but still in contact with the generator plate or may be spaced apart a short distance so that surface tension holds the fluid in the chamber. The generator plate may have a relatively small maximum amplitude when vibrated which is less than an average separation distance between the lip and the generator plate or less than a minimum separation distance between the lip and the generator plate.

The enclosure may also be shaped to cooperate with the generator plate to avoid capillary feed near the openings in the generator plate. To this end, the enclosure may be spaced apart from the generator plate so that at least 75%, at least 95%, or all of the openings are spaced at least 0.014 from the nearest part of the enclosure. The enclosure may also shaped so that all of the fluid can reach the openings of the generator plate in a short period of time. The enclosure may be configured with an internal surface shape in contact with the fluid such that at least 75%, at least 95%, or even all, of the internal surface is no more than 0.060 inch, or no more than 0.040 inch, from a nearest of the plurality of openings of the generator plate. Stated another way, the enclosure has an internal surface shaped such that the chamber is formed with at least 75%, at least 95%, or all, of the internal surface having direct line of sight to at least one of the openings of the generator plate. The inner surface of the enclosure may be hydrophobic over at least 70% of the inner surface in contact with fluid.

The lip may be biased against the generator plate with a modest force to prevent the fluid from escaping while not overly dampening vibrations. The lip may exert a force of no more than 3 gram-f on the vibrating element measured in the direction of a central axis of the vibrating element. The lip may also apply a spring load to the generator plate so that minor displacements due to temperature, pressure or shock from an impact (dropped) can be accommodated. The spring load may also help to address manufacturing tolerances which affect the load applied by the lip to the generator plate. The lip may exert a spring load on the generator plate with an average spring constant of no more than 60 gram-f/mm for displacements up to 0.050 mm. The enclosure itself may be resilient with a wall of the enclosure having a tapered portion with a relatively thin wall to provide flexibility. The tapered portion of the wall and has a ratio of radial displacement to longitudinal displacement of at least 1 to 3, at least 1 to 2 and may be at least 1 to 1. Stated another way, the tapered portion also extends radially with respect to the open end of the enclosure for at least half of an effective radius of the open end of the enclosure. The lip and/or the vibrating element may have a PTFE coating adjacent to the other to reduce friction therebetween. The coating(s) may extending around at least 270 degrees when viewed along the central axis.

The enclosure may allow air into to replace ejected fluid through the openings of the generator plate and/or between the lip and the generator plate and may include no dedicated vent opening. The maximum amplitude may be somewhat small which permits air to enter the chamber while still preventing fluid from escaping from the chamber. The enclosure to generator plate interface defines an enclosed border (which may be defined by either the generator plate or the enclosure) which is somewhat larger than the extent of the openings with an excess area which extends radially outwardly at least 0.3 times the effective radius of the enclosed feed area.

The enclosure may include a wall opening through the wall which exposes the chamber through the wall. The wall opening. The wall opening extends through the wall to expose the chamber through the wall without permitting fluid to escape while permitting air to enter when fluid is ejected. The wall opening has a longitudinal dimension measured from the lip in the direction of the central axis and a radial dimension measured in a radial direction relative to the central axis. The enclosure also has an internal wall with a side facing the openings in the generator plate. The longitudinal dimension of the wall opening is at least 80% of a separation distance between the generator plate and the side of the enclosure facing the openings. The radial dimension of the wall opening may be no more than 10%, or no more than 5%, of a circumference of the lip.

The wall opening tapers as it extends proximally away from the lip. The wall opening extends from the lip proximally and a circumferential dimension of the wall opening tapers down as the wall opening extends proximally from the lip. The wall opening tapers so that a tapered shape is oriented in the direction of the fluid inlet to the enclosure when viewed along the central axis. The wall opening may also extend through the frustoconical portion of the wall and may extend proximally from the lip for at least 80% of the length of the frustoconical portion.

The fluid may be delivered rapidly and at relatively high velocity and pressure to encourage all of the fluid to gather in the chamber. The total downstream volume of the fluid path from a pump or valve which isolates the chamber may be sized somewhat larger than the volume to permit the fluid to move within the enclosure somewhat and coalesce into a single droplet due to surface tension. The volume of the fluid may be 40%-70% of the total downstream volume.

The enclosure may also split the fluid flow into at least two (and may be three, four or more) inlets to the chamber. Each of the inlets directs the fluid at a sidewall before being directed at the plurality of openings of the generator plate. The enclosure has a main inlet which directs the flow in a direction within 30 degrees of the central axis while the inlets to the chamber are oriented 60-90 degrees from the central axis and directed at the sidewall. The enclosure may be an integrally formed structure which defines the chamber.

The pump may have a first part and a second part which reciprocate between a stored position, to a forward stroke position and back to the stored position in a single cycle. A cavity is formed between the two parts in which the fluid is drawn and subsequently expelled into the chamber. An air make-up chamber may also be coupled to the pump to force air into a fluid reservoir during each cycle to actively vent the fluid reservoir.

It is understood that the droplet delivery device may have be separated into reusable and disposable portions in innumerable different combinations. For example, the enclosure may form part of a reusable device together with the ejector mechanism, or may be disposable with the reservoir without departing from the scope of the invention.

In certain aspects, the stream of droplets may be generated by devices described herein in a controllable distribution of sizes, each distribution having an average droplet size as defined in the claims. In certain embodiments, the average droplet size is greater than 15 microns, about 15 microns to about 100 microns, about 20 microns to about 100 microns, greater than 20 microns to about 100 microns, about 20 microns to about 80 microns, about 25 microns to about 75 microns, about 30 microns to about 60 microns, about 35 microns to about 55 microns, *etc.*

The device may also deliver the fluid at relatively high velocity which helps the fluid "stick" to the target tissue which, in this case, is corneal tissue resulting in direct corneal surface coating with the fluid. Eye droppers, on the other hand, do not have the ability to target a particular area of the eye and deliver the fluid at high velocity. Eye drops are large enough to migrate and seep into undesirable areas after delivery. In this regard, the droplets may have an average initial ejecting velocity of about 0.5 m/s to about 20 m/s, *e.g.,* about 0.5 m/s to about 15 m/s, about 0.5 m/s to about 10 m/s, about 1 m/s to about 10 m/s, about 4 m/s to about 12 m/s, about 1 m/s to about 5 m/s, about 1 m/s to about 4 m/s, at least about 2 m/s, at least about 3 m/s, at least about 4 m/s, at least about 5 m/s, *etc.* As used herein, the ejecting size and the ejecting initial velocity are the size and velocity of the droplets when the droplets leave the ejector plate. The stream of droplets directed at a target will result in deposition of a percentage of the mass of the droplets including their composition onto the desired location.

In certain aspects of the disclosure, the ejector devices will eject droplets without substantial evaporation, entrainment of air, or deflection off a target surface *(e.g.,* the surface of an eye), which facilitates consistent dosing. Average ejecting droplet size and average initial ejecting velocity are dependent on factors including fluid viscosity, surface tension, ejector plate properties, geometry, and dimensions, as well as operating parameters of the piezoelectric actuator including its drive frequency. In some implementations, about 60% to about 100%, about 65% to about 100%, about 75% to about 100%, about 80% to about 100%, about 85% to about 100%, about 90% to about 100%, about 95% to about *100%, etc.,* of the ejected mass of droplets are deposited on the surface of the eye, such deposition being repeatable independent of operating and use conditions.

The direction of flow of the stream of droplets may be horizontal, or any direction a user chooses to aim the actuation mechanism during use. In certain aspects, the device may be oriented substantially horizontal, e.g., within 5° of horizontal, 10° of horizontal, 15° of horizontal, 20° of horizontal, 25° of horizontal, etc., and may aid the user in aiming the device. For example, a light, mirror or other visual alignment feature may be used as is known in the art. Horizontal delivery with alignment assistance may also improve ease of use and repeatability compared to an eyedropper which has no "aiming" mechanism and the drop is too large to target just corneal tissue. In this regard, it has been found that the same amount of drug with horizontal targeted corneal delivery and coating achieves higher pharmacodynamic effect compared to same amount of drug given in an eyedropper.

Droplet performance is generally related to particle diameter. Without intending to be limited, ejected droplets are slowed to a stop by air drag *(i.e.,* stopping distance of the ejected droplets). Ejected droplets also fall vertically due to gravity. After a short acceleration time, the droplets reach terminal velocity where the drag force equals the force of gravity. The ejected droplets may carry air along with them, which creates an entrained airstream, which aids to then carry the ejected droplets beyond the calculated stopping distance. However, increased levels of entrained air may cause the ejected droplets to flow across an impact surface *(e.g.,* an eye surface) because the entrained airflow must turn 90 degrees at such a surface. Small, ejected droplets (e.g., droplets having an average diameter less than about 17 microns, less than about 15 microns, *etc.)* are carried along the surface of the eye by the airstream and may not impact the surface. Contrasted to this, larger ejected droplets create less entrained air than an equivalent mass of smaller droplets, and have enough momentum to impact the surface. The ejected droplet stopping distance is a measure of this effect.

Many factors, including those described herein, can influence the desired dosage. Once the desired dosage is determined, and also if needed, desired frequency, such doses can be delivered. Frequency of dosing can vary by number of times, periodicity or both.

The term "therapeutically effective" amount refers to an amount of an active agent used to treat, ameliorate, prevent, or eliminate the identified ophthalmic condition (e.g., disease or disorder), or to exhibit a detectable therapeutic or preventive effect. The effect can be detected by, for example, chemical markers, antigen levels, or time to a measurable event, such as morbidity or mortality. The precise effective amount for a subject will depend upon the subject's body weight, size, and health; the nature and extent of the condition; and the therapeutic or combination of therapeutics selected for administration. Effective amounts for a given situation can be determined by routine experimentation that is within the skill and judgment of the clinician. Any of the agents can be provided in an effective amount.

In an aspect, the concentration of an active ingredient in a medicament is measured as a percentage of the active ingredient in solution. In an aspect, the concentration of active ingredient ranges from about 0.0001% to about 5%. In another aspect, the concentration of active ingredient in a medicament ranges from about 0.05% to about 1%. In other aspects, the concentration of active ingredient ranges from about 0.1%, about 0.2%, about 0.3%, about 0.4%, about 0.5%, about 0.75%, about 1%, about 1.5%, about 2%, about 2.5%, about 3%, about 4%, and about 5% measured as a weight percentage of the solution. In certain embodiments, the concentration of active ingredient ranges from about 0.5 % by weight, at least 0.8 % by weight, 1% or more by weight, 2% or more by weight, 3% or more by weight, 4% or more by weight, 0.5 to 1.2% by weight, 0.5 to 2% by weight, 0.5 to 3% by weight, 0.5 to 4% by weight. However, given the lower dosing amounts afforded by the methods of the present disclosure, higher concentrations may be used depending on the intended use.

### EXAMPLE

A multi-center, double-masked, placebo-controlled study evaluating the safety and efficacy of relatively high concentration (1% to 4%) pilocarpine solution administered in small volume micro-dose for the temporary improvement of near vision in subjects with presbyopia is described herein.

### STUDY OBJECTIVE

The primary objective of this study is to evaluate the safety and efficacy of pilocarpine solution (1% to 4%) administered via a piezoelectric droplet delivery device, as described herein, for the temporary improvement of functional near vision in presbyopia as compared a placebo.

### STUDY PRODUCTS

Product(s), Dosage(s) and Mode(s) of Administration:
- Pilocarpine 1% (~8µl) ophthalmic solution self-administered bilaterally as a microdose using the piezoelectric droplet delivery device
- Pilocarpine 2% (~8µl) ophthalmic solution self-administered bilaterally as a microdose using the piezoelectric droplet delivery device
- Pilocarpine 4% (~8µl) ophthalmic solution self-administered bilaterally as a microdose using the piezoelectric droplet delivery device

Placebo, Dosage and Mode of Administration: Vehicle of Pilocarpine solution self-administered by the droplet device.

### STUDY POPULATION

Up to 120 volunteer participants will be enrolled at 4-6 study sites and a minimum of 96 subjects will be randomized to study drug self-administration at Treatment Visit 1 in order to complete follow-up on 84 or more subjects.

### STUDY DESIGN

This trial is a double-masked, active-controlled, cross-over superiority study evaluating 4 study drugs administered by the piezoelectric droplet delivery device. The drugs evaluated are:
- Pilocarpine 1% ophthalmic solution,
- Pilocarpine 2% ophthalmic solution,
- Pilocarpine 4% ophthalmic solution, and
- Vehicle Solution (placebo)

Volunteer participants will be screened for study eligibility during a Screening Visit and enrolled after signing the study-specific informed consent form (ICF). Subjects meeting all inclusion/exclusion criteria will be scheduled for 4 treatment visits, which must occur at least 3 days, but no more than 10 days apart. At each treatment visit, baseline measurements will be taken, then 1 spray of the assigned study drug will be self-administered to both eyes (OU). Afterwards, efficacy and safety assessments will be performed at specific time intervals. Study drug administration for each subject will be equally randomized to one of 12 possible sequences of study drugs and, by the completion of the 4^{th} treatment visit, each subject will have received all four study drugs. At several timepoints through 4 hours post-medication administration, the key study assessments (DCNVA, DCIVA and pupil size) will be performed; the primary efficacy endpoint (DCNVA) will be evaluated at 3 hours.

The cross-over design will randomize subjects to one of 12 possible treatment sequences based on an orthogonal Latin square design with 3 4x4 squares. Jones, B., and M. G. Kenward, 2015. Design and analysis of cross-over trials. CRC Press. pp 140. This design is balanced for carryover in that each treatment follows every other treatment twice.

This study will be double-masked. There will be no differences in the presentation of study drug administered and all study personnel conducting ophthalmic assessments will be masked to study drug assignment. Personnel who observe study drug administration by subjects on a given day will not be allowed to perform post-drug administration ophthalmic assessments on that day. Subjects who are missing assessments at any of the scheduled study timepoints or are otherwise unevaluable will not be replaced.

The use of any systemic medication that might affect pupil dilation or constriction will be recorded with the goal that no changes to the frequency, dosage, or route of administration will be made during the course of the study unless medically necessary.

### Inclusion Criteria

- Subjects eligible for study participation must meet each criterion listed below. Ocular criteria must be met for both eyes.
- Male or female, 40-60 years of age at the time of the Screening Visit
- Subjective complaint of poor near vision that impacts activities associated with daily living and requiring regular use of reading glasses or bifocals
- In need of near addition power of +1.0 D through +2.0 D based on near acuity refraction at Screening Visit
- Monocular DCNVA between 20/60-20/100 (inclusive) at both the Screening and Baseline Visits
- Female subjects must be 1-year postmenopausal, surgically sterilized, or if of childbearing potential, have a negative serum pregnancy test at the Day 1 Visit and agree to use an acceptable form of contraception throughout the study. *Acceptable methods include the use of at least one of the following: intrauterine (intrauterine device), hormonal (oral, injection, patch, implant, ring), barrier with spermicide (condom, diaphragm), or abstinence.*
- Myopia Refractive error (MRSE) ≥ -0.50 D and ≤+1.00D.
- Best-corrected distance visual acuity (BCDVA) of 20/20 or better in each eye
- Understand protocol requirements and able to provide signed informed consent prior to participation in any study-related procedures
- Ability to return for all study treatment visits.

### Exclusion Criteria

Subjects with any of the following diseases, surgeries or conditions are ineligible for study participation. Subjects may not participate if either eye meets any of the ocular exclusion criteria.
- Irregular astigmatism or astigmatism ≥1.00 D
- Diagnosis of any type of glaucoma or ocular hypertension
- Narrow iridocorneal angles, history of angle-closure glaucoma, or previous iridotomy
- Known pilocarpine allergy or contraindication to pilocarpine
- Allergy to benzalkonium chloride
- Clinically significant abnormality of the cornea, lens, central retina, ciliary body or iris including any of the following:
   - Irregularly-shaped pupil secondary to ocular trauma or congenital defect
   - Abnormal findings on dilated fundus exam documented within 3 months of the Screening Visit, or a known history of retinal detachment or clinically significant retinal disease
   - History of traumatic iritis or hyphema
   - History of traumatic mydriasis or angle recession
   - History of neurogenic pupil disorder (e.g., Horner's syndrome, third cranial nerve palsy, Adie's pupil, Argyl Robertson syndrome, etc.)
   - History of iris atrophy
   - History of chronic or acute uveitis
   - History of heterochromia
   - History of iris - cornea apposition/touch
- External ocular inflammation within 30 days of Screening Visit
- Ocular surgery or laser treatment of any kind prior to the Screening Visit including iris surgery of any kind (e.g., iridotomy, iridectomy, coreoplasty)
- Current or prior history of manifest strabismus, amblyopia, or nystagmus
- Preexisting neurological diagnoses (e.g. cerebral palsy) or genetic syndrome (e.g. Down syndrome)
- Use of temporary or permanent punctal plugs or history of punctal cautery in one or both eyes
- Pregnancy or lactation, or intending to become pregnant within the 3 months following the Screening Visit
- Mesopic pupillometry measurements of < 4.0 mm
- Participation in any interventional study of an investigational drug or device within 30 days prior to the Screening Visit or at any time during the study period
- Lid squeezer (e.g., blepharospasm)
- History of RGP lens use within 4 weeks of screening and unwilling or unable to discontinue use of RGP lenses during the entire study
- Unwilling or unable to discontinue use of soft contact lenses at all treatment visits
- History of drug or alcohol abuse within 1 year prior to the Screening Visit
- Current or prior history of congenital heart anomaly, valve disease or other cardiac disease
- Current active eye disease for which topical or systemic ophthalmic medication is necessary, except for dry eye disease managed using artificial tears (AT). *AT's must be discontinued on the day of each treatment visit.*
- Presence of a severe/serious ocular condition, or any other unstable medical condition that, in the Investigator's opinion, may preclude study treatment and/or follow-up
- Presence of disabling arthritis or limited motor coordination that would limit self- handling of the droplet device per the judgment of the Investigator
- Immediate family member of study staff who is designated to perform study evaluations or procedures.

### Efficacy Endpoints

Mesopic (2-3 cd/m²), high contrast, binocular DCNVA (distance-corrected near visual acuity) will be assessed at baseline, 30 minutes, 90 minutes, 3 hours, and 4 hours. The primary efficacy endpoint will be assessed at 3 hours.

### Primary

Proportion of subjects gaining 15 letters or more in mesopic, high contrast, binocular DCNVA (distance-corrected near visual acuity) at 3 hours post-treatment compared to baseline.

### Exploratory Outcomes: Distance Corrected Near Visual Acuity (DCNVA) (40 cm)

- Proportion of subjects gaining 3 lines (15 letter) or more in mesopic, high contrast DCNVA (monocular) at 3 hours post-treatment compared to baseline DCNVA (Primary endpoint is binocular DCNVA)
- Proportion of subjects gaining 3 lines (15 letter) or more in mesopic, high contrast DCNVA (monocular and binocular) at other timepoints (Primary endpoint is binocular DCNVA)
- Proportion of subjects gaining 2 lines (10 letter) or more in mesopic, high contrast DCNVA (monocular and binocular) at each timepoint
- Mean Change from Baseline in DCNVA (monocular and binocular) at each timepoint
- Distribution of DCNVA at each timepoint
- Time from baseline to maximal mean DCNVA

### Exploratory Outcomes: Distance Corrected Intermediate Visual Acuity (DCIVA) (40 cm)

- Proportion of subjects gaining 3 lines (15 letter) or more in mesopic, high contrast DCIVA (binocular) at 3 hours post-treatment compared to baseline DCIVA
- Proportion of subjects gaining 3 lines (15 letter) or more in mesopic, high contrast DCIVA (binocular) at other timepoints
- Proportion of subjects gaining 3 lines (15 letter) or more in mesopic, high contrast DCIVA (monocular) at each timepoint
- Proportion of subjects gaining 2 lines (10 letter) or more in mesopic, high contrast DCIVA (monocular and binocular) at each timepoint
- Mean Change from Baseline in DCIVA (monocular and binocular) at each timepoint
- Distribution of DCIVA at each timepoint
- Time from baseline to maximal mean DCIVA

### Exploratory (Other)

- Change from Baseline in pupil diameter in mesopic conditions and appearance/roundness (assessed at 30 minutes, 90 minutes, 3 hours, and 4 hours)
- Patient preference and usability feedback
- Change from baseline in Defocus Curve assessed at 3 hours

### Efficacy Analyses

### Primary

The primary analysis will be stratified by baseline DCNVA (above and below 20/80) in this presbyopic study population.

The proportion of subjects gaining 15 letters or more of binocular DCNVA at 3 hours post-dose will be summarized by treatment group, baseline DCNVA stratum, and visit (including across all visits) with descriptive statistics.

For each baseline stratum, a conditional logistic regression will be fitted to the binary response data. The following covariates will be included in the logistic model: treatment, period, first order carryover, iris color (dark or light), baseline DCNVA, age (continuous), add power, and use of systemic medications at screening that could cause pupil dilation/constriction (yes or no). To condition on the subject effect the subjects will be included as strata (in SAS). The Firth method will be used to allow estimation to proceed even if there are no responders in the placebo group. Since the design is balanced for carryover there is no need to introduce a random subject effect unless loss to follow-up is substantial. Jones and Kenward, 2015, Section 6.2.2.4.

Pairwise comparisons of Pilocarpine 1% (~8µl) ophthalmic solution (PC_{1%}), Pilocarpine 2% (~8µl) ophthalmic solution (PC_{2%}), and Pilocarpine 4% (~8µl) ophthalmic solution (PC_{4%}) with placebo (PLC) will be based on the fitted model.

Within each baseline stratum the following three hypotheses will be tested:
H₀: log Odds Ratio for PCₓ vs PLC <= 0
H_{A}: log Odds Ratio for PCₓ vs PLC > 0
Where x is one of {1%, 2%, 4%}

The study success criterion is that at least one of the three null hypotheses for the primary outcome will be rejected for at least one baseline stratum. To maintain the type-I experiment-wise error rate at 0.025 (one-sided):
- A Bonferroni correction will be applied across baseline strata, and then
- within each stratum, the significance level for each of the three comparisons of Pilocarpine versus Placebo will be selected based on Dunnett's (1955) procedure.

For each pairwise comparison within each stratum, the expected marginal mean (least squares mean) will be computed for the log odds ratio, that is, the three contrasts between each of the pilocarpine arms and the placebo arm, based on the logistic regression model. In constructing these contrasts, all other covariates in the model will be set at their mean observed value. Confidence intervals will be constructed for each contrast using the likelihood profile method from the Firth logistic regression.

The level for the confidence intervals will be set to control the experiment-wise one- sided type-I error at 0.025 as described above. For each contrast, if the lower bound of the confidence interval for the log odds ratio is greater than 0.0 then the null hypothesis for that contrast will be rejected.

If the study success criterion is met then the primary analysis will be repeated for the fellow eyes.

As a sensitivity analysis a global model that includes both baseline strata will be fitted, eliminating covariates that are not significant at the p=0.10 level.

### Exploratory outcomes: Distance Corrected Near Visual Acuity (DCNVA), Distance Corrected Intermediate Visual Acuity (DCIVA), and pupil diameter

The following will all be analyzed the same manner as the primary outcome:
- Proportion of subjects gaining 3 lines (15 letter) or more in mesopic, high contrast DCNVA (monocular) at 3 hours post-treatment compared to baseline DCNVA
- Proportion of subjects gaining 3 lines (15 letter) or more in mesopic, high contrast DCNVA (monocular and binocular) at other timepoints.
- Proportion of subjects gaining 2 lines (10 letter) or more in mesopic, high contrast DCNVA (monocular and binocular) at each timepoint
- Proportion of subjects gaining 3 lines (15 letter) or more in mesopic, high contrast DCIVA (binocular) at 3 hours post-treatment compared to baseline DCIVA
- Proportion of subjects gaining 3 lines (15 letter) or more in mesopic, high contrast DCIVA (binocular) at other timepoints
- Proportion of subjects gaining 3 lines (15 letter) or more in mesopic, high contrast DCIVA (monocular) at each timepoint
- Proportion of subjects gaining 2 lines (10 letter) or more in mesopic, high contrast DCIVA (monocular and binocular) at each timepoint

The following will be analyzed using a generalized additive model (GAM) with a (4 df) smooth term for measurement time (estimated separately for each stratum and treatment arm), and fixed effects for treatment, treatment, period, carryover, iris color, baseline DCNVA/DCIVA (logMAR), age, and add power. To allow for correlation between measurement times, subject will be included as a random effect in the model.

- Mean Change from Baseline in logMAR DCNVA and DCIVA (monocular and binocular) by treatment at each timepoint
- Time from baseline to maximum mean logMAR DCNVA and DCIVA by treatment

The fitted smooth function of time will be used to predict the mean change from baseline logMAR DCNVA and DCIVA, to determine the maximum mean change in logMAR DCNVA and DCIVA by treatment, and to estimate the time of the maximum for each treatment.

The following will be analyzed using nonparametric quantile smoothing:
- Distribution of logMAR DCNVA and DCIVA at each timepoint
- Distribution of pupil diameter at each timepoint
- Distribution of change from baseline pupil diameter at each timepoint

The distribution of logMAR DCNVA, DCIVA and pupil diameter at each time point will be characterized by 5df quantile smoothing splines applied across time. Splines will be fit to the following quantiles: 0.025, 0.10, 0.25, 0.50, 0.75, 0.90, 0.975. Note that a 5df spline will smoothly interpolate the observed quantiles at each time point.

The smooth spline fit to the median will be used to characterize the change in logMAR through time:
- Start, end, and total time during which the median improvement in binocular DCNVA and DCIVA is 15 letters (0.3 logMAR units) or greater, by stratum and treatment group.
- Total area under the logMAR improvement curve from time 0 through 3 hours, by stratum and treatment group.

For each of these measures confidence intervals will be constructed using a clustered bootstrap procedure (with individual subjects as clusters).

### Safety Outcomes

- Slit-lamp examination and fundus findings
- Mean change in IOP at Hour 4?
- Rates of ocular and non-ocular adverse events (AE)
- Mean Change from Baseline in best corrected distance visual acuity (monocular and binocular) measured at Hour 3 and Hour 4
- Mean Change from Baseline in uncorrected distance visual acuity (monocular and binocular) measured at Hour 3 and Hour 4
- Ocular Comfort Assessment (Visual Analog Scale) following instillation of study drug and 30 minutes after installation

### Sample Size

The study design is a diagram-balanced Latin square with 4 treatments, 4 periods, 3 blocks and 12 sequences. Jones and Kenward, 2015, Section 4.2.1. This design is balanced for all first-order carryover effects, and allows estimation using a fixed effects ANOVA model. To maintain the balance for each of the two DCNVA baseline strata the overall sample size will need to be a multiple of 24 (12 per stratum, see Table 1).

**Table 1: Diagram-balanced Latin square with 4 treatments, 4 periods, 3 blocks and 12 sequences.**

| **Block** | **Sequence Number** | **Period 1** | **Period 2** | **Period 3** | **Period 4** |
|---|---|---|---|---|---|
| **1** | **1** | A | B | C | D |
| | **2** | B | A | D | C |
| | **3** | C | D | A | B |
| | **4** | D | C | B | A |
| **2** | **5** | A | D | B | C |
| | **6** | B | C | A | D |
| | **7** | C | B | D | A |
| | **8** | D | A | C | B |
| **3** | **9** | A | C | D | B |
| | **10** | B | D | C | A |
| | **11** | C | A | B | D |
| | **12** | D | B | A | C |

Sample sizes were calculated using simulations for selected null and alternative scenarios. Note that due to the very small (p=0.02) expected response rate in the placebo arm, normality is not plausible, thus the Dunnett adjustment was also calculated by simulation.

Table 2 below shows the calculated sample sizes (evaluable study eyes) that provide

>96% power (to reject at least one of the three comparisons for at least one of the two strata) for a range of proportions in the placebo (p0) and treated groups (p1, p2, and p3). The power calculations assume there are two strata, with alpha of 0.0125 allocated to each stratum, and a Dunnet adjustment applied within the stratum for the comparison of the three active treatment arms to the placebo control. Within each stratum, the power is set at 80%, resulting in an overall power of about 96%.

Note that:
- This calculation, based on a simple comparison of proportions, should be conservative if the covariates in the logistic model are predictive of outcome,
- since the sample size is rounded up to the nearest multiple of 24 and also allows for 5% loss to follow-up the actual power may be higher,
- this calculation assumes independence of the outcomes within subject over the 4 treatments. If there is a positive correlation, then the power will be increased since the within-subject variance for the difference will be smaller.

The total required number of randomized subjects is then calculated to allow for approximately 5% loss-to-follow-up prior to the primary efficacy analysis, and then rounded up to the nearest 12 per stratum. Based on an expected responder proportion of 2% in the Placebo arm and expected responder proportions of 20%, 25%, and 30% in the pilocarpine arms, randomization of at least 96 subjects (48 per stratum) is required to achieve the required power, after adjusting for multiple comparisons to a single control. This should result in at least 84 evaluable study eyes (42 per stratum) at the conclusion of the study.

To account for screen failures, the study will screen a maximum of 120 subjects, continuing until 48 subjects per stratum have been randomized.

Note that because both eyes of each subject are treated during the study, safety information will be available for approximately 192 eyes from the 96 subjects who are randomized and initiate treatment with microdosed pilocarpine (1%, 2%, or 4%) administered with the piezoelectric droplet delivery device.

**Table 2. Required sample sizes per stratum and overall for >95% power for significance of either PC_{1%} vs PLC, PC_{2%} vs PLC, or PC_{4%} vs PLC. P0, P1, P2 P3 are the assumed responder rates for PLC, PC_{1%}, PC_{2%}, and PC_{4%}. The study is a 4-treatment 4-period crossover with 2 baseline strata, assuming no carryover effect. N evaluable are the number of evaluable subjects required to provide the specified power. N randomized assumes a 5% loss to follow-up and balance within strata (so, is rounded up to nearest multiple of 24).**

| **H1:p0** | **H1:p1** | **H1:p2** | **H1:p3** | **n evaluable per stratum** | **n evaluable both strata** | **Total n randomized (5% LTFU, balanced)** |
|---|---|---|---|---|---|---|
| 0.02 | 0.10 | 0.15 | 0.20 | 61 | 122 | 144 |
| 0.02 | 0.15 | 0.20 | 0.25 | 42 | 84 | 96 |
| 0.02 | 0.20 | 0.25 | 0.30 | 30 | 60 | 72 |
| 0.02 | 0.25 | 0.30 | 0.35 | 24 | 48 | 72 |
| 0.02 | 0.30 | 0.35 | 0.40 | 19 | 38 | 48 |

The present invention has been described with reference to certain embodiments, however, various modifications may be made without departing from the features and aspects of the invention.

## Claims

1. A composition comprising pilocarpine for use in the treatment of presbyopia in an eye of a subject, wherein the composition is delivered to the eye as a micro-dose stream of droplets, wherein:
the micro-dose stream of droplets including the composition comprising pilocarpine is generated via a piezoelectric droplet delivery device, wherein the micro-dose stream of droplets has an average ejected droplet diameter greater than 15 microns.

2. The composition for use of claim 1, wherein the composition comprises pilocarpine as a single active agent.

3. The composition for use of claim 1 or claim 2, for treating or alleviating one or more symptoms of presbyopia in the subject in need thereof; optionally for the temporary improvement of functional near vision in presbyopia, as compared to placebo.

4. The composition for use of any one of claims 1 to 3, wherein the composition comprises pilocarpine at a concentration of 2 % by weight.

5. The composition for use of any one of claims 1 to 3, wherein the composition comprises pilocarpine at a concentration of concentration of 1% to 4% by weight.

6. The composition for use of any one of claims 1 to 5, wherein the volume of the micro-dose stream of droplets delivered to the eye of the subject is 3 microliters to 15 microliters.

7. The composition for use of any one of claims 1 to 5, wherein the volume of the micro-dose stream of droplets delivered to the eye of the subject is less than 10 microliters.

8. The composition for use of any one of claims 1 to 5, wherein the volume of the micro-dose stream of droplets delivered to the eye of the subject is 3 microliters to 8 microliters.

9. The composition for use of any of claims 1 to 8, wherein the micro-dose stream of droplets has an average initial ejecting velocity of at least 3 m/s, optionally of 4 m/s to 12 m/s.

10. The composition for use of any of claims 1 to 9, wherein the micro-dose stream of droplets is delivered to the eye of the subject in less than 80 ms.

11. The composition for use of any of claims 1 to 10, wherein the micro-dose stream of droplets has an average ejected droplet diameter of 20 microns to 100 microns.

12. The composition for use of any of claims 1 to 11, wherein the piezoelectric droplet delivery device is oriented within 25° of horizontal during generation and delivery of the micro-dose stream of droplets.

13. The composition for use of any of claims 1 to 12, wherein the piezoelectric droplet delivery device comprises an ejector mechanism, the ejector mechanism comprising a generator plate and a piezoelectric actuator, wherein the generator plate includes a plurality of openings formed through its thickness; and wherein the piezoelectric actuator is operable to directly or indirectly oscillate the generator plate, at a frequency to generate the stream of droplets.

14. The composition for use of claim 13, wherein the piezoelectric droplet delivery device further comprises a fluid enclosure system having a lip positioned adjacent the generator plate, the lip unattached to the generator plate and in contact with the generator plate or the lip spaced apart from the generator plate,
wherein the generator plate vibrates at a maximum amplitude that is less than an average separation distance between the lip and the generator plate or less than a minimum separation distance between the lip and the generator plate.

15. The composition for use of claim 13, wherein the piezoelectric droplet delivery device further comprises a fluid enclosure system having a lip biased against the generator plate with a force measured in a direction of a central axis of a vibrating element.

## Patentansprüche

1. Zusammensetzung, die Pilocarpin zur Verwendung bei der Behandlung von Presbyopie in einem Auge eines Subjekts umfasst, wobei die Zusammensetzung dem Auge als ein mikrodosierter Tröpfelstrom zugeführt wird, wobei:
der mikrodosierte Tröpfelstrom, der die Zusammensetzung einschließt, die Pilocarpin umfasst, über eine piezoelektrische Tröpfchenzufuhrvorrichtung erzeugt wird, wobei der mikrodosierte Tröpfelstrom einen durchschnittlichen ausgestoßenen Tröpfchendurchmesser von mehr als 15 Mikrometern aufweist.

2. Zusammensetzung zur Verwendung nach Anspruch 1, wobei die Zusammensetzung Pilocarpin als einen einzelnen Wirkstoff umfasst.

3. Zusammensetzung zur Verwendung nach Anspruch 1 oder Anspruch 2 zur Behandlung oder Linderung eines oder mehrerer Symptome von Presbyopie bei dem Subjekt, das dessen bedarf; optional zur vorübergehenden Verbesserung des funktionellen Nahsehens bei Presbyopie im Vergleich zu Placebo.

4. Zusammensetzung zur Verwendung nach einem der Ansprüche 1 bis 3, wobei die Zusammensetzung Pilocarpin in einer Konzentration von 2 Gew.-% umfasst.

5. Zusammensetzung zur Verwendung nach einem der Ansprüche 1 bis 3, wobei die Zusammensetzung Pilocarpin in einer Konzentration von 1 Gew.-% bis 4 Gew.-% umfasst.

6. Zusammensetzung zur Verwendung nach einem der Ansprüche 1 bis 5, wobei das Volumen des mikrodosierten Tröpfelstroms, der dem Auge des Subjekts zugeführt wird, 3 Mikroliter bis 15 Mikroliter beträgt.

7. Zusammensetzung zur Verwendung nach einem der Ansprüche 1 bis 5, wobei das Volumen des mikrodosierten Tröpfelstroms, der dem Auge des Subjekts zugeführt wird, weniger als 10 Mikroliter beträgt.

8. Zusammensetzung zur Verwendung nach einem der Ansprüche 1 bis 5, wobei das Volumen des mikrodosierten Tröpfelstroms, der dem Auge des Subjekts zugeführt wird, 3 Mikroliter bis 8 Mikroliter beträgt.

9. Zusammensetzung zur Verwendung nach einem der Ansprüche 1 bis 8, wobei der mikrodosierte Tröpfelstrom eine durchschnittliche anfängliche Ausstoßgeschwindigkeit von mindestens 3 m/s, optional von 4 m/s bis 12 m/s aufweist.

10. Zusammensetzung zur Verwendung nach einem der Ansprüche 1 bis 9, wobei der mikrodosierte Tröpfelstrom dem Auge des Subjekts in weniger als 80 ms zugeführt wird.

11. Zusammensetzung zur Verwendung nach einem der Ansprüche 1 bis 10, wobei der mikrodosierte Tröpfelstrom einen durchschnittlichen ausgestoßenen Tröpfchendurchmesser von 20 Mikrometern bis 100 Mikrometern aufweist.

12. Zusammensetzung zur Verwendung nach einem der Ansprüche 1 bis 11, wobei die piezoelektrische Tröpfelzufuhrvorrichtung während Erzeugung und Zufuhr des mikrodosierten Tröpfelstroms innerhalb von 25° der Horizontalen ausgerichtet ist.

13. Zusammensetzung zur Verwendung nach einem der Ansprüche 1 bis 12, wobei die piezoelektrische Tröpfchenzufuhrvorrichtung einen Ausstoßmechanismus umfasst, wobei der Ausstoßmechanismus eine Erzeugerplatte und ein piezoelektrisches Stellglied umfasst, wobei die Erzeugerplatte eine Vielzahl von Öffnungen einschließt, die durch ihre Dicke hindurch ausgebildet ist; und wobei das piezoelektrische Stellglied betätigbar ist, um die Erzeugerplatte direkt oder indirekt mit einer Frequenz zu oszillieren, um den Tröpfelstrom zu erzeugen.

14. Zusammensetzung zur Verwendung nach Anspruch 13, wobei die piezoelektrische Tröpfchenzufuhrvorrichtung ferner ein Fluidumschließungssystem mit einer Lippe umfasst, die angrenzend an die Erzeugerplatte positioniert ist, wobei die Lippe nicht an der Erzeugerplatte befestigt ist und in Kontakt mit der Erzeugerplatte steht oder wobei die Lippe von der Erzeugerplatte beabstandet ist,
wobei die Erzeugerplatte mit einer maximalen Amplitude vibriert, die kleiner als ein durchschnittlicher Trennungsabstand zwischen der Lippe und der Erzeugerplatte oder kleiner als ein minimaler Trennungsabstand zwischen der Lippe und der Erzeugerplatte ist.

15. Zusammensetzung zur Verwendung nach Anspruch 13, wobei die piezoelektrische Tröpfchenzufuhrvorrichtung ferner ein Fluidumschließungssystem umfasst, das eine Lippe aufweist, die gegen die Erzeugerplatte mit einer Kraft vorgespannt ist, die in einer Richtung einer Mittelachse eines vibrierenden Elements gemessen wird.

## Revendications

1. Composition comprenant de la pilocarpine destinée à être utilisée dans le traitement de la presbyopie dans un œil d'un sujet, ladite composition étant administrée à l'œil sous la forme d'un flux de microdoses de gouttelettes :
ledit flux de microdoses de gouttelettes comprenant la composition comprenant de la pilocarpine étant généré par l'intermédiaire d'un dispositif piézoélectrique de distribution de gouttelettes, ledit flux de microdoses de gouttelettes ayant un diamètre moyen de gouttelettes éjectées supérieur à 15 microns.

2. Composition destinée à être utilisée de la revendication 1, ladite composition comprenant de la pilocarpine en tant qu'agent actif unique.

3. Composition destinée à être utilisée de la revendication 1 ou la revendication 2, pour traiter ou soulager un ou plusieurs symptômes de la presbyopie chez le sujet en ayant besoin ; éventuellement pour l'amélioration temporaire de la vision de près fonctionnelle dans la presbyopie, par rapport au placebo.

4. Composition destinée à être utilisée de l'une quelconque des revendications 1 à 3, ladite composition comprenant de la pilocarpine à une concentration de 2 % en poids.

5. Composition destinée à être utilisée de l'une quelconque des revendications 1 à 3, ladite composition comprenant de la pilocarpine à une concentration de concentration de 1 % à 4 % en poids.

6. Composition destinée à être utilisée de l'une quelconque des revendications 1 à 5, ledit volume du flux de microdoses de gouttelettes administré à l'œil du sujet étant de 3 microlitres à 15 microlitres.

7. Composition destinée à être utilisée de l'une quelconque des revendications 1 à 5, ledit volume du flux de microdoses de gouttelettes administré à l'œil du sujet étant inférieur à 10 microlitres.

8. Composition destinée à être utilisée de l'une quelconque des revendications 1 à 5, ledit volume du flux de microdoses de gouttelettes administré à l'œil du sujet étant de 3 microlitres à 8 microlitres.

9. Composition destinée à être utilisée de l'une quelconque des revendications 1 à 8, ledit flux de microdoses de gouttelettes ayant une vitesse d'éjection initiale moyenne d'au moins 3 m/s, éventuellement de 4 m/s à 12 m/s.

10. Composition destinée à être utilisée de l'une quelconque des revendications 1 à 9, ledit flux de microdoses de gouttelettes étant administré à l'œil du sujet en moins de 80 ms.

11. Composition destinée à être utilisée de l'une quelconque des revendications 1 à 10, ledit flux de microdoses de gouttelettes ayant un diamètre moyen de gouttelettes éjectées de 20 microns à 100 microns.

12. Composition destinée à être utilisée de l'une quelconque des revendications 1 à 11, ledit dispositif piézoélectrique d'administration de gouttelettes étant orienté à 25° maximum par rapport à l'horizontale pendant la génération et l'administration du flux de microdoses de gouttelettes.

13. Composition destinée à être utilisée de l'une quelconque des revendications 1 à 12, ledit dispositif piézoélectrique de distribution de gouttelettes comprenant un mécanisme d'éjection, le mécanisme d'éjection comprenant une plaque génératrice et un actionneur piézoélectrique, ladite plaque génératrice comprenant une pluralité d'ouvertures formées dans son épaisseur ; et ledit actionneur piézoélectrique pouvant fonctionner pour faire osciller directement ou indirectement la plaque génératrice, à une fréquence pour générer le flux de gouttelettes.

14. Composition destinée à être utilisée de la revendication 13, ledit dispositif piézoélectrique de distribution de gouttelettes comprenant en outre un système d'enceinte de fluide ayant une lèvre positionnée adjacente à la plaque génératrice, la lèvre n'étant pas fixée à la plaque génératrice et étant en contact avec la plaque génératrice ou la lèvre étant espacée de la plaque génératrice,
ladite plaque génératrice vibrant à une amplitude maximale qui est inférieure à une distance de séparation moyenne entre la lèvre et la plaque génératrice ou inférieure à une distance de séparation minimale entre la lèvre et la plaque génératrice.

15. Composition destinée à être utilisée de la revendication 13, ledit dispositif piézoélectrique d'administration de gouttelettes comprenant en outre un système d'enceinte de fluide ayant une lèvre sollicitée contre la plaque génératrice avec une force mesurée dans une direction d'un axe central d'un élément vibrant.
